# EUROPÄISCHE PATENTSCHRIFT

(11) **EP 0 791 569 B1**
(45) Veröffentlichungstag und Bekanntmachung des Hinweises auf die Patenterteilung: **17.11.1999**
(21) Anmeldenummer: 97102133.2
(22) Anmeldetag: 11.02.1997
(51) Int. Cl.: C07B 55/00, C07C 209/68

(54) **Verfahren zur Racemisierung optisch aktiver Amine**
Process for the razemization of optically active amines
Procédé pour la racémisation d'amines optiquement actives

(30) Priorität: 20.02.1996 DE 19606124
(43) Veröffentlichungstag der Anmeldung: 27.08.1997
(73) Patentinhaber: BASF AKTIENGESELLSCHAFT, 67056 Ludwigshafen (DE)
(72) Erfinder: Ditrich, Klaus, Dr., 67161 Gönnheim (DE)

(56) Entgegenhaltungen:
- EP-A- 0 489 682
- DATABASE WPI Section Ch, Week 9538 Derwent Publications Ltd., London, GB; Class B05, AN 95-290356 XP002031534 & JP 07 188 120 A (TORAY IND INC) , 25.Juli 1995

## Beschreibung

Die vorliegende Erfindung betrifft ein Verfahren zur Racemisierung optisch aktiver Amine.

Bei einer Reihe von Wirkstoffsynthesen werden optisch aktive Amine als Zwischenprodukte eingesetzt. Dabei wird in der Regel nur ein optischer Antipode benötigt, während der andere Antipode als Nebenprodukt anfällt. Um diese Syntheseverfahren wirtschaftlicher zu gestalten, ist es sinnvoll, den nicht-gewünschten Antipoden zu racemisieren und aus diesem racemischen Gemisch erneut den gewünschten Antipoden herzustellen.

Aus DE 28 51 039 ist ein Verfahren zur Racemisierung von 1-Arylalkylaminen durch Reaktion mit Wasserstoff/Raney-Cobalt bekannt. Unter diesen Bedingungen finden jedoch auch unerwünschte Nebenreaktionen wie Dehalogenierung am Arylrest statt, so daß dieses Verfahren für viele Arylalkylamine nicht geeignet ist.

In EP 489 682 wird ein Verfahren zur Racemisierung von optisch aktiven 1-Arylalkylaminen beschrieben, bei dem man die Amine mit Metallalkanolaten in Dimethylsulfoxid umsetzt. Dieses Verfahren ist jedoch für einen Einsatz im technischen Maßstab nicht gut geeignet, da es teure Reagenzien benötigt, einen zusätzlichen Neutralisationsschritt erfordert und keine kontinuierliche Fahrweise gestattet.

In JP 07188120 wird die Racemisierung von optisch aktiven 1-(Halophenyl)ethylaminen beschrieben, die folgende Verfahrensschritte umfaßt:
(i) Kondensation des 1-(Halophenyl)ethylamins mit einem Acetophenon,
(ii) Erhitzen des Kondensationsprodukts in Gegenwart von Alkalimetallalkoxid und
(iii) Hydrolyse.

Dieses Verfahren ist ebenfalls ein diskontinuierliches Verfahren, bei dem eine Reihe von zusätzlichen Aufarbeitungsschritten (Ansäuern, Extraktion des Acetophenons, Freisetzen des Amins durch Base) nötig sind.

Es bestand daher die Aufgabe, ein Verfahren zur Racemisierung von optisch aktiven Arylalkylaminen bereitzustellen, das die Nachteile der o.g. Verfahren nicht besitzt.

Gegenstand der Erfindung ist ein Verfahren zur Racemisierung von optisch aktiven Aminen der Formel (I), wobei Ar für ein gegebenenfalls substituiertes Aryl und R für Alkyl steht, bei dem
a) (I) mit dem Keton (II), in dem die Reste Ar und R die gleiche Bedeutung wie bei (I) besitzen, zum Kondensationsprodukt (III) umgesetzt wird, 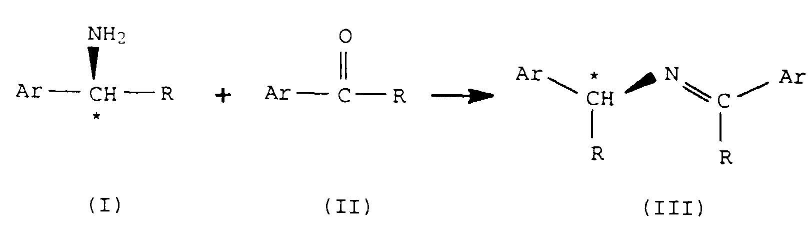
b) (III) durch Behandlung mit Base racemisiert wird, 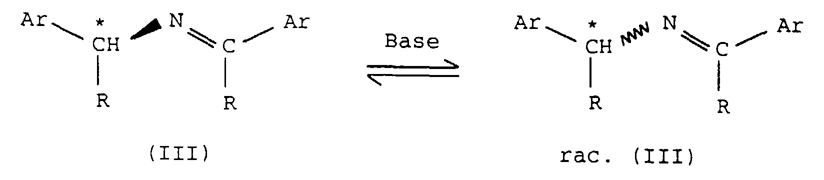
c) aus racemischem (III) durch Umsatz mit optisch aktivem (I) das Arylalkylamin (I) als Racemat freigesetzt wird. 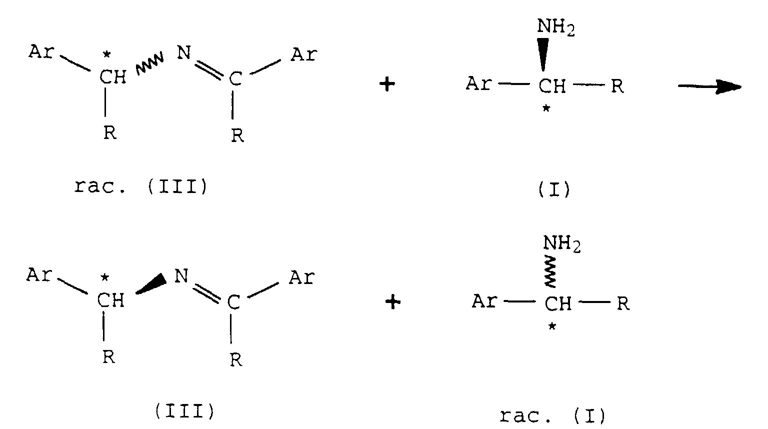 C* soll ein asymmetrisches Kohlenstoffatom darstellen.

Als optisch aktive Amine (I) können für das erfindungsgemäße Verfahren die reinen R- oder S-Formen von (I) oder auch Mischungen aus R-und S-Form von (I) in beliebigen Mengenverhältnissen eingesetzt werden.

In der allgemeinen Formel für (I) steht Ar für ein gegebenenfalls ein- bis fünffach gleich oder verschiedenartig substituiertes Aryl, vorzugsweise Phenyl oder Naphthyl. Die möglichen Substituenten sind bevorzugt aus der Gruppe Halogen, Nitro, Cyano, offenkettiges oder zyklisches Alkyl, Alkoxy, Alkylthio ausgewählt. Die genannten Alkyl-, Alkoxy- und Alkylthiosubstituenten können selbst wiederum substituiert sein durch beispielsweise ein oder mehrere Halogenatome.

Besonders bevorzugt werden Verbindungen (I), In denen Ar für ein mit elektronenziehenden Resten substituiertes Phenyl steht. Ganz besonders bevorzugt sind einfach substituierte Halogenphenyle, beispielsweise 2-, 3- oder 4-Chlorphenyl.

Der Rest R steht in der allgemeinen Formel für (I) für ein gegebenenfalls substituiertes geradkettiges oder verzweigtes Alkyl, vorzugsweise mit 1 bis 10, insbesondere mit 1 bis 5 C-Atomen. Ganz besonders bevorzugt ist R = Methyl.

Die Verbindungen (II) sind Arylketone, bei denen die Reste Ar und R identisch mit den jeweiligen Resten der Amine (I) sind.

Die Ketone (II) werden mit den Aminen (I) in einer Kondensationsreaktion umgesetzt (a). Diese Umsetzung kann mit üblichen Lösungsmitteln oder auch lösungsmittelfrei durchgeführt werden. Bevorzugt wird das erfindungsgemäße Verfahren ohne Lösungsmittel ausgeführt. Die Umsetzung erfolgt üblicherweise bei einer Temperatur von 0 bis 300, bevorzugt von 20 bis 200°C. Dabei entsteht als Kondensationsprodukt (III) eine Schiff'sche Base, die durch Zugabe einer Base racemisiert wird (b).

Als Base geeignet sind Alkoholate wie Natrium- oder Kaliummethanolat, Natrium- oder Kaliumethanolat, Natrium- oder Kalium-t-butanolat oder tertiäre Amine, insbesondere Diazabicyclooctan (Dabco), Diazabicyclononan (DBN), Diazabicycloundecan (DBU) und Tri-n-octylamin.

Aus dem Kondensationsprodukt (III) wird durch Umsatz mit optisch aktivem (I) das Amin (I) in racemischer Form freigesetzt (c).

Die Menge an zugesetztem Keton (II) kann in weiten Bereichen variiert werden, da (II) im Verfahren wieder zurückgewonnen wird. Wird eine schnelle Racemisierung gewünscht, ist es angebracht, den Anteil an (II) zu erhöhen; andererseits kann man mit geringeren Mengen an (II) auskommen, wenn man dafür die Racemisierungszeit verlängert. Bevorzugt wird (II) in einer Menge von 1 bis 10 Mol.-% bezogen auf (I), eingesetzt.

Das erfindungsgemäße Verfahren wird in der Regel unter Normaldruck durchgeführt. Es ist jedoch auch unter verminderten oder erhöhten Druckverhältnissen ausführbar.

Nach Ablauf der Racemisierungsreaktion wird das racemische Amin (I) mit üblichen Methoden gewonnen, beispielsweise durch Destillation aus dem Reaktionsgemisch.

Der Destillationsrückstand braucht dabei nicht verworfen zu werden, sondern kann für weitere Racemisierung verwendet werden, indem man diesen Rückstand mit weiterem optisch aktivem I umsetzt und die Racemisierungsreaktion erneut durchläuft.

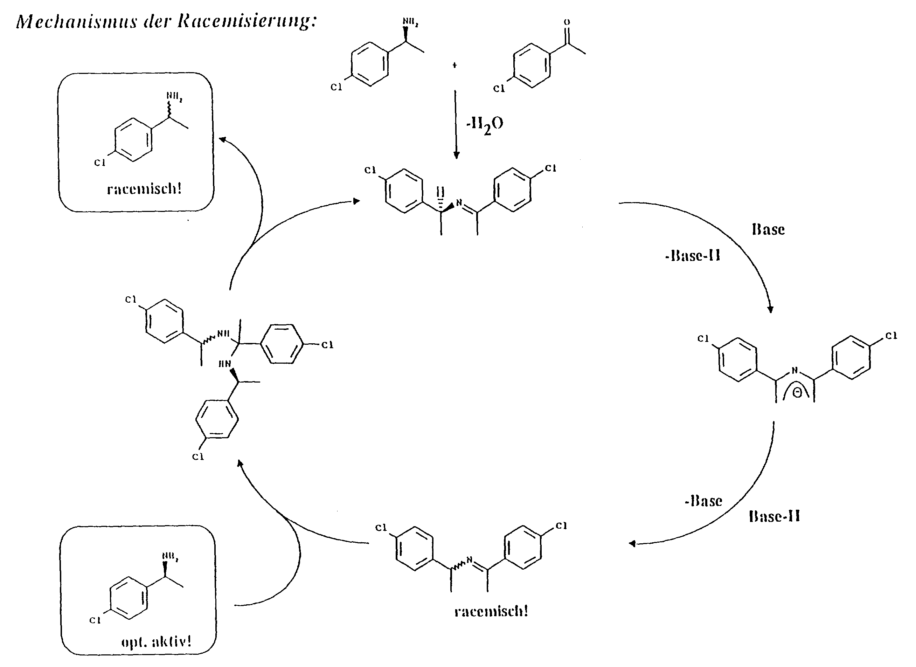

Dieses Verfahren eignet sich daher besonders zum kontinuierlichen Betrieb, bei dem wie in Abb. 1 dargestellt, verfahren wird.

### Beispiel

### Racemisierung von S-1-(4-Chlorphenyl)ethylamin

10,0 g S-1-(4-Chlorphenyl)ethylamin (ee = 100 %) wurden mit 1,0 g 4-Chloracetophenon versetzt, zwei Stunden auf 100°C erhitzt und anschließend mit 1,0 g Diazabicycloundecan (DBU) versetzt. Die Temperatur wurde auf 190°C erhöht. Nach 80 Stunden war das Amin vollständig racemisiert. Nach Destillation erhielt man 8,0 g racemisches 1-(4-Chlorphenyl)ethylamin.

Der Destillationsrückstand wurde mit optisch aktivem 1-(4-Chlorphenyl)ethylamin versetzt und erneut zur Racemisierung eingesetzt.

## Patentansprüche

1. Verfahren zur Racemisierung von optisch aktiven Aminen der Formel (I), wobei Ar für ein gegebenenfalls substituiertes Aryl und R für Alkyl steht, bei dem
a) (I) mit dem Keton (II), in dem die Reste Ar und R identisch mit den jeweiligen Resten der Amine (I) sind, zum Kondensationsprodukt (III) umgesetzt wird, 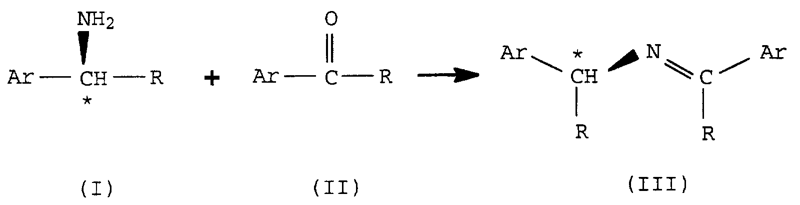
b) (III) durch Behandlung mit Base racemisiert wird, 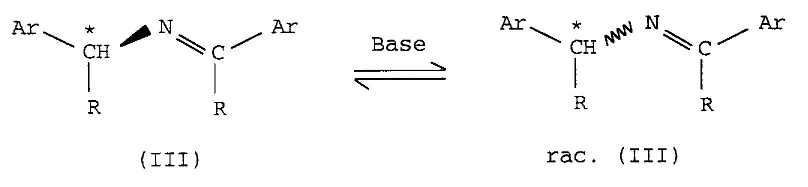
c) aus racemischem (III) durch Umsatz mit optisch aktivem (I) das Arylalkylamin (I) als Racemat freigesetzt wird.

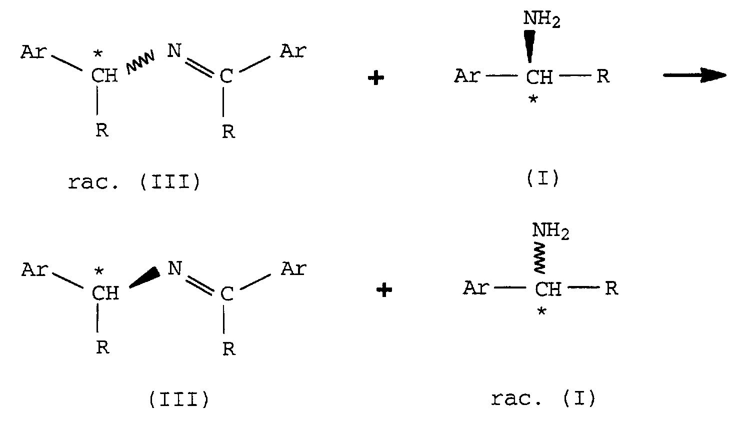

2. Verfahren nach Anspruch 1, dadurch gekennzeichnet, daß in der Formel (I) Ar für ein gegebenenfalls ein- bis fünffach gleich oder verschiedenartig durch Halogen, Nitro, Cyano, offenkettiges oder zyklisches Alkyl, Alkoxy, Alkylthio substituiertes Phenyl oder Naphthyl steht, und R für ein gegebenenfalls substituiertes geradkettiges oder verzweigtes Alkyl mit 1 bis 10 C-Atomen steht.

3. Verfahren nach Anspruch 2, dadurch gekennzeichnet, daß Ar für Chlorphenyl und R für Methyl steht.

4. Verfahren nach Anspruch 1 bis 3, dadurch gekennzeichnet, daß das Verfahren kontinuierlich durchgeführt wird.

## Claims

1. A process for the racemization of optically active amines of the formula (I), where Ar is an unsubstituted or substituted aryl and R is alkyl, in which
a) (I) is reacted with the ketone (II) in which Ar and R are identical to the respective radicals of the amines (I), to give the condensation product (III), 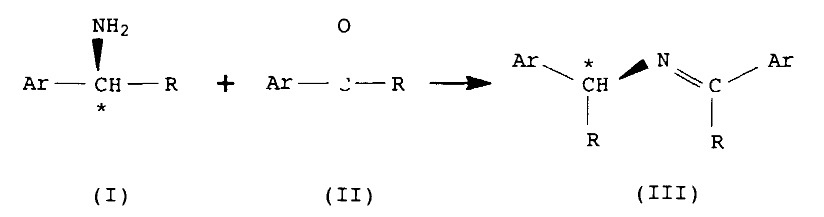
b) (III) is racemized by treatment with base, 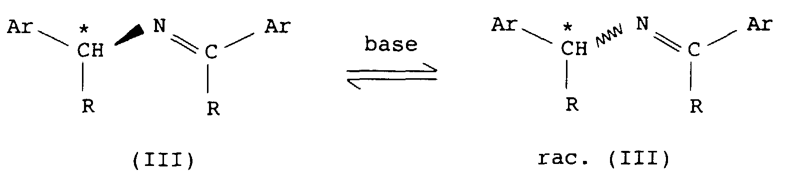
c) the arylalkylamine (I) is liberated as racemate from racemic (III) by reaction with optically active (I).

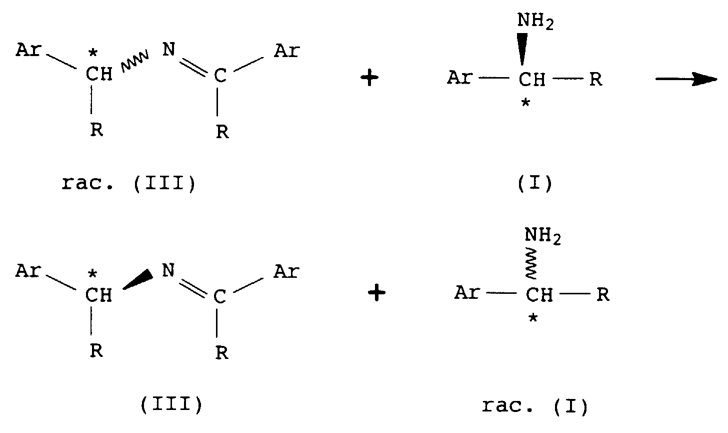

2. A process as claimed in claim 1, wherein Ar in formula (I) is phenyl or naphthyl which is unsubstituted or substituted once to five times, identically or differently, by halogen, nitro, cyano, open-chain or cyclic alkyl, alkoxy or alkylthio, and R is an unsubstituted or substituted straight-chain or branched alkyl with 1 to 10 carbon atoms.

3. A process as claimed in claim 2, wherein Ar is chlorophenyl and R is methyl.

4. A process as claimed in any of claims 1 to 3, which is carried out continuously.

## Revendications

1. Procédé pour racémiser des amines de formule I dans laquelle Ar représente un groupe aryle éventuellement substitué et R un groupe alkyle, à l'état d'isomères optiques, selon lequel
**a)** on fait réagir I avec la cétone II pour laquelle Ar et R sont identiques aux groupes correspondants du composé I, ce qui donne le produit de condensation III 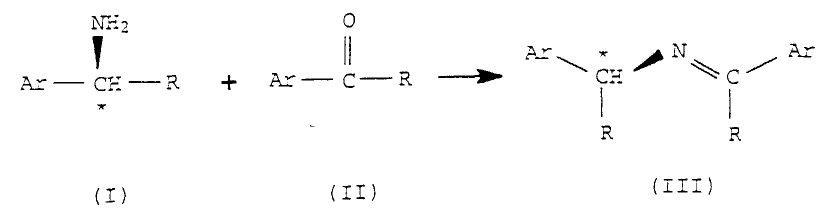
**b)** on racémise III par traitement à l'aide d'une base 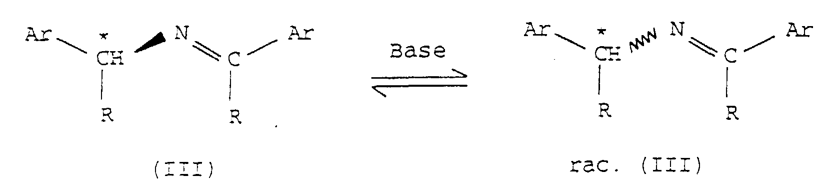
**c)** à partir du racémique III, par réaction avec I à l'état d'isomère optique, on libère l'arylalkylamine I à l'état de racémate.

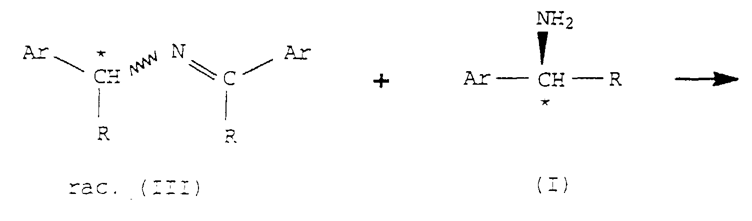

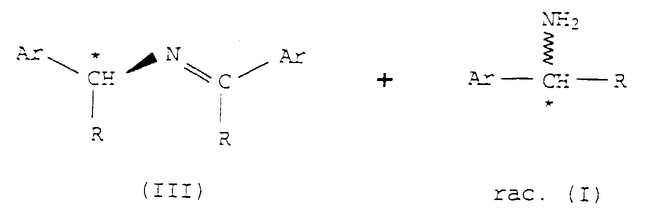

2. Procédé selon la revendication 1, caractérisé par le fait que, dans la formule I, Ar représente un groupe phényle ou naphtyle portant éventuellement un à cinq substituants identiques ou différents choisis parmi les halogènes, les groupes nitro, cyano, alkyle acyclique ou cyclique, alcoxy, alkylthio, et R représente un groupe alkyle à chaîne droite ou ramifiée en C1-C10 éventuellement substitué.

3. Procédé selon la revendication 2, caractérisé par le fait que Ar représente un groupe chlorophényle et R un groupe méthyle.

4. Procédé selon les revendications 1 à 3, caractérisé par le fait que l'on opère en continu.
